Europäisches Patentamt

European Patent Office

Office européen des brevets

(11) Veröffentlichungsnummer: **0 678 495 A2**

(12) **EUROPÄISCHE PATENTANMELDUNG**

(21) Anmeldenummer: **95105579.7**

(22) Anmeldetag: **13.04.95**

(51) Int. Cl.[6]: **C07C 17/395**, C07C 19/14

(30) Priorität: **20.04.94 DE 4413658**

(43) Veröffentlichungstag der Anmeldung:
**25.10.95 Patentblatt 95/43**

(84) Benannte Vertragsstaaten:
**AT BE CH DE DK ES FR GB GR IE IT LI LU NL PT SE**

(71) Anmelder: **HOECHST AKTIENGESELLSCHAFT**

**D-65926 Frankfurt am Main (DE)**

(72) Erfinder: **Raab, Klaus, Dr.
Ortlehnerstrasse 9
D-84508 Burgkirchen (DE)**

(54) **Verfahren zur Reinigung von Perfluoralkylbromiden oder Perfluoralkylendibromiden.**

(57) Perfluoralkanmono- und -dibromide können durch Behandeln mit einem primären oder sekundären Alkohol und einer Base von Verunreinigungen, insbesondere von den entsprechenden Iodiden, befreit werden.

Perfluorierte Bromalkanverbindungen, beispielsweise Perfluorhexylbromid und insbesondere Perfluoroctylbromid, werden unter anderem für medizinische Zwecke eingesetzt, wobei sie zum Beispiel intravenös oder gastrointestinal appliziert werden. Sie dienen beispielsweise als Kontrastmittel bei der Untersuchung mit Röntgenstrahlen oder mit Ultraschall, zur Erkennung von Tumoren, zur Organperfusion und in wäßriger Emulsion als Blutersatzstoff. Für diese Anwendungsgebiete werden außergewöhnlich hohe Reinheitsanforderungen gestellt, insbesondere ist die Anwesenheit von Iodverbindungen unerwünscht.

Aus der US-A 5 175 380 ist ein Verfahren zur Entfernung von Verunreinigungen aus einer Verbindung oder Verbindungen der Formel $X(CF_2)_nBr$, in der bedeuten $X = F$, $(CF_3)_2CF$ oder Br und n = eine Zahl von 2 bis 16, bekannt, das dadurch gekennzeichnet ist, daß das zu reinigende Gemisch mit einer elektromagnetischen Strahlung im Wellenlängenbereich von 230 bis 500 nm bestrahlt, während oder nach der Bestrahlung mit mindestens einem der folgenden Mittel: Aktivkohle, einem feinteiligen Feststoff aus der Reihe Cu, CuI, Ag, Mg, Zn, Al, Mn, Fe, Co, Ni und einem Alkalimetallborhydrid, Chlor, Brom, Sauerstoff, wäßrige Lösungen von $H_2O_2$, anderen anorganischen peroxidischen Verbindungen oder Alkalimetallsalzen mit folgenden Anionen: $SO_3^{2-}$, $S_2O_3^{2-}$, $PO_3^{3-}$, $NO_2^-$, $CO_3^{2-}$, $HCO_3^-$, $BrO_3^-$, $ClO_3^-$, wäßrige oder alkoholische Lösungen von Alkalimetallhydroxiden, -iodiden, -alkoholaten oder niedrigen aliphatischen Alkoholen in Kontakt gebracht, nach Beendigung der Behandlung das Mittel abgetrennt und das zu reinigende Gemisch, falls erforderlich, mit Wasser gewaschen und destilliert wird.

Dieses bekannte Verfahren ist sehr leistungsfähig, insbesondere wenn die Umsetzung mit Brom stattfindet. Es ist allerdings wegen der erforderlichen Apparatur und insbesondere der anzuwendenden Strahlung recht teuer. Es wurde nun gefunden, daß man einen ähnlichen Reinigungseffekt auch dadurch erzielen kann, daß man die störenden Verunreinigungen - im wesentlichen neben den bereits genannten Iodverbindungen auch ungesättigte Verbindungen - in von den gewünschten Produkten gut abtrennbare Verbindungen überführt. Diese Überführung gelingt dadurch, daß man die verunreinigten Bromalkanverbindungen mit einem primären oder sekundären Alkohol und einer Base in Kontakt bringt und die reinen Bromalkanverbindungen abtrennt.

Die Erfindung betrifft somit ein Verfahren zur Reinigung von Perfluorbromalkanen der Formel (I)

$$X\text{-}C_nF_{2n}\text{-}Br \qquad (I)$$

in der X Fluor oder Brom und n eine Zahl von 2 bis 16 bedeuten, das dadurch gekennzeichnet ist, daß man die zu reinigende Verbindung der Formel (I) mit einem primären oder sekundären Alkohol und einer Base unter guter Durchmischung in Kontakt bringt und die Alkohol-Base-Phase von der Verbindung der Formel (I) abtrennt. Bevorzugte Ausführungsformen der Erfindung werden im folgenden näher erläutert.

Bevorzugt sind Ausgangsmaterialien der Formel (II)

$$Y\text{-}(CF_2)_m\text{-}Br \qquad (II)$$

in der Y Fluor, Brom oder die Gruppe $(CF_3)_2CF$- und m eine Zahl von 2 bis 13 bedeuten.

Das erfindungsgemäße Verfahren kann auf rohe Bromfluoralkane angewendet werden, wie sie aus den entsprechenden perfluorierten Iodalkanverbindungen mit elementarem Brom oder Bromiden gewonnen werden. Der Fachmann wird hier im Einzelfall abwägen, ob er diese Rohprodukte unmittelbar dem erfindungsgemäßen Reinigungsverfahren zuführt oder ob er diese Rohprodukte, wenn sie zum Beispiel mehr als etwa 5 % Iodverbindungen enthalten, zunächst - beispielsweise destillativ - auftrennt und dadurch Ausgangsmaterialien für das erfindungsgemäße Verfahren gewinnt, die über 95 %, vorzugsweise über 98 %, an Verbindungen der Formel (I) enthalten.

Soweit bei dem erfindungsgemäßen Verfahren perfluorierte Iodalkanverbindungen umgesetzt werden, dient der eingesetzte Alkohol als Reduktionsmittel, der entsprechend zum korrespondierenden Aldehyd oder Keton oxidiert wird. Hierbei wurde festgestellt, daß beim Einsatz von Methanol und Natriummethylat zum Teil Kohlenmonoxid entwickelt wird, so daß in diesem Falle entsprechende Vorkehrungen zu treffen sind. Im Falle der niedermolekularen Alkohole sind deshalb Verbindungen mit 2 bis 4 C-Atomen bevorzugt.

Eine besonders bevorzugte Ausführungsform der Erfindung besteht darin, daß als Alkohol ein Polyalkylenglykol oder Polyalkylenglykolmonoether eingesetzt wird. Bevorzugt sind die Polyethylenglykole und Polypropylenglykole. Besonders bevorzugt sind Polyethylenglykole mit einer mittleren Molmasse von 200 bis 2000. Vorteilhaft sind modifizierte Polyalkylenglykole, wie sie nach dem Verfahren der EP-B 166 958 erhältlich sind, da diese Produkte auch bei Raumtemperatur flüssig und somit auch bei niedrigen Temperaturen gut mit den anderen Komponenten durchmischbar sind.

Der besondere Vorteil beim Einsatz der Polyglykole und ihrer Derivate besteht darin, daß die durch Oxidation gebildeten Nebenprodukte gut von den Verbindungen der Formel (I) abtrennbar sind. Je nach den

2

Anforderungen an die Reinheit der Verbindung der Formel (I) genügt die Phasentrennung und gegebenenfalls eine Wasserwäsche oder eine einfache Destillation, wobei die Nebenprodukte im Sumpf verbleiben.

Das Massenverhältnis der zu reinigenden Verbindung der Formel (I) und des primären oder sekundären Alkohols richtet sich nach dem Grad der Verunreinigung und kann nötigenfalls durch einen einfachen Vorversuch bestimmt werden. Es liegt im allgemeinen zwischen 20 : 1 und 1 : 1.

Die einzusetzende Base ist bevorzugt ein Alkalimetallalkoholat, Alkalimetalloxid oder Alkalimetallhydroxid, insbesondere Kaliumhydroxid oder Natriumhydroxid. Das Molverhältnis von perfluorierter Iodalkanverbindung und Base ist ebenfalls abhängig vom Grad der Verunreinigung und kann nötigenfalls durch einen einfachen Vorversuch bestimmt werden. Es liegt im allgemeinen zwischen 1 : 3 und 1 : 50.

Das erfindungsgemäße Verfahren wird im allgemeinen bei Temperaturen von -10 bis +150 °C, vorteilhaft von +10 bis +80 °C, durchgeführt.

Bei einer bevorzugten Ausgestaltung der Erfindung wird die Base in dem Alkohol gelöst und der zu reinigenden Verbindung der Formel (I) in dem Maße zugesetzt, daß der pH-Wert in der Alkohol-Base-Phase stets im alkalischen Bereich gehalten wird. Im Falle einer Nachdosierung kann die Zugabe der Base oder der Lösung der Base im Alkohol mehrmals oder kontinuierlich erfolgen.

Bei dem erfindungsgemäßen Reinigungsverfahren entstehen aus den perfluorierten Iodalkanverbindungen die entsprechenden Hydroperfluoralkane. Diese Verbindungen sind chemisch und physiologisch inert und stören deshalb bei den üblichen Einsatzzwecken nicht. Sofern ihre Abtrennung erforderlich ist, ist diese aufgrund des unterschiedlichen Siedepunkts durch einfache Destillation möglich.

Die Überführung dieser Iodverbindungen in die Hydroverbindungen ist an sich bekannt, beispielsweise aus H. Meinert et al., J. Fluorine Chem. 59 (1992), Seiten 379 bis 385. Nach den dort angegebenen Daten müßte Methanol ein ideales Reinigungsmittel sein, da - bei den Reinsubstanzen - die Iodverbindungen zu über 90 % umgesetzt werden, während die Bromverbindungen nicht angegriffen werden. Bei den Ausgangsmaterialien für das erfindungsgemäße Reinigungsverfahren liegen jedoch kleine Mengen an Iodverbindungen neben ganz überwiegenden Anteilen Bromverbindungen vor. Unter diesen Verhältnissen wird auch die Bromverbindung nennenswert angegriffen, weshalb Methanol für das erfindungsgemäße Verfahren nicht bevorzugt ist.

Ungesättigte Verbindungen und gegebenenfalls weitere Verunreinigungen werden bei dem erfindungsgemäßen Verfahren ebenfalls in gut abtrennbare Produkte überführt. Sofern erforderlich, kann das Reaktionsgemisch einem oder mehreren der folgenden Reinigungsschritte unterworfen werden: Waschen mit Wasser, Behandeln mit Aktivkohle sowie Destillation. Der Fachmann wird anhand einfacher Vorversuche je nach den Anforderungen an das Reinprodukt die entsprechenden Reinigungsschritte oder gleichwirkende Maßnahmen auswählen beziehungsweise kombinieren.

Das erfindungsgemäße Verfahren wird in den folgenden Beispielen näher erläutert. Prozentangaben sind Masseprozent, sofern keine anderen Angaben gemacht sind.

Polyethylenglykole sind durch ihre mittlere Molmasse in Form einer nachgestellten Ziffer charakterisiert, der Zusatz "PR" bedeutet, daß es sich um modifizierte, bei Zimmertemperatur flüssige Produkte nach der EP-B 166 958 handelt.

Beispiel 1

In einem 50-ml-Glaskolben, ausgestattet mit Rückflußkühler, Magnetrührfisch und Innenthermometer, werden 30 g einer Mischung bestehend aus 99,3 % Perfluoroctylbromid der Formel $CF_3(CF_2)_7Br$, 0,63 % Perfluoroctyliodid der Formel $CF_3(CF_2)_7I$ und 0,017 % Perfluorheptyliodid der Formel $CF_3(CF_2)_6I$ zusammen mit einer zuvor unter Stickstoff hergestellten Lösung von 0,33 g Natriumhydroxid in 7,5 g Polyethylenglykol 600 PR vorgelegt. Der zweiphasige, zunächst farblose Kolbeninhalt wird unter Stickstoff 8 Stunden bei 80 °C unter guter Durchmischung gerührt. Nach Abkühlen auf 30 °C wird die obere, dunkelbraune Polyethylenglykol-600-PR-Phase von der sich rasch absetzenden unteren, klaren und farblosen Perfluoroctylbromid-Phase im Scheidetrichter abgetrennt. Der pH-Wert der Polyethylenglykol-600-PR-Phase liegt nach der Umsetzung noch im alkalischen Bereich bei etwa pH 10. Die Perfluoroctylbromid-Phase wird insgesamt dreimal mit je 10 ml destilliertem Wasser bei Raumtemperatur ausgeschüttelt. Die kapillargaschromatographische Untersuchung des rohen Perfluoroctylbromids ergibt folgende Zusammensetzung:

0,015 % $CF_3(CF_2)_6H$

1,4 % $CF_3(CF_2)_7H$

< 0,001 % $CF_3(CF_2)_6I$ } (Gehalt unter der

< 0,001 % $CF_3(CF_2)_7I$ } Bestimmungsgrenze)

98,5 % $CF_3(CF_2)_7Br$

26,2 g des geruchlosen Perfluoroctylbromids werden bei Normaldruck über eine Spaltrohrkolonne fraktioniert. Der bei 960 mbar Druck und bei 141 °C Kopftemperatur abgenommene Hauptlauf ist Perfluoroctylbromid mit einer Reinheit von über 99,9 %.

Beispiele 2 bis 6

Beispiel 1 wird wiederholt, wobei statt Polyethylenglykol 600 PR zum Teil andere Polyglykole eingesetzt werden. Die von Beispiel 1 abweichenden Reaktionsbedingungen und die Ergebnisse der kapillargaschromatographischen Untersuchungen sind in der nachfolgenden Tabelle 1 aufgeführt.

## Tabelle 1

| Bei-spiel | Polyglykol/ Menge [g] | Base/ Menge [g] | Reaktions-temperatur [°C] | Zusammensetzung des rohen Perfluoroctylbromides vor Destillation | | | | |
|---|---|---|---|---|---|---|---|---|
| | | | | $C_7F_{15}H$ | $C_8F_{17}H$ | $C_7F_{15}I$ | $C_8F_{17}I$ | $C_8F_{17}Br$ |
| 2 | Polyethylen-glykol 600 PR/ 15,0 | NaOH/ 0,55 | 80 | 0,017 % | 2,6 % | < 0,001 % | < 0,001 % | 97,3 % |
| 3 | Polyethylen-glykol 600 PR/ 7,5 | NaOH/ 0,31 | 60 | 0,012 % | 1,3 % | < 0,001 % | 0,0012 % | 98,6 % |
| 4 | Polyethylen-glykol 600 PR/ 7,5 | NaOH/ 0,42 | 24 | 0,011 % | 0,76 % | < 0,001 % | 0,0025 % | 99,1 % |
| 5 | Polyethylen-glykol 1000 PR/ 5,9 | NaOH/ 0,24 | 80 | 0,021 % | 1,9 % | < 0,001 % | < 0,001 % | 98,0 % |
| 6 | Polyethylen-glykol 600/ 13,5 | NaOH/ 0,28 | 60 | 0,011 % | 0,75 % | < 0,001 % | 0,0082 % | 99,1 % |

Beispiel 7

In einem 50-ml-Glaskolben, ausgestattet mit Rückflußkühler, Magnetrührfisch und Innenthermometer, werden 15 g Polyethylenglykol 600 und 0,3 g Natriumhydroxid unter Stickstoff bei Raumtemperatur gerührt, bis alles Natriumhydroxid gelöst ist. Bei Raumtemperatur werden unter Rühren 30 g einer Mischung bestehend aus 97,9 % Perfluoroctylbromid, 0,11 % Perfluorheptyliodid und 1,9 % Perfluoroctyliodid auf einmal Zugegeben. Die Temperatur steigt ohne Heizung kurzzeitig auf 40 °C und der zweiphasige Kolbeninhalt wird 8 Stunden bei 28 °C unter Stickstoff gerührt. Zur Verfolgung des Reaktionsfortschritts werden nach 1 Stunde und nach 4 Stunden Proben gezogen. Die obere, dunkelbraune, bei Raumtemperatur etwas zähflüssige Polyethylenglykol-600-Phase wird im Scheidetrichter von der unteren, fast farblosen und klaren Perfluoroctylbromid-Phase abgetrennt und die Perfluoroctylbromid-Phase dreimal mit je 10 ml destilliertem Wasser ausgeschüttelt. Die kapillargaschromatographische Untersuchung des rohen Perfluoroctylbromids vor der Destillation ergibt folgende Zusammensetzung:
0,074 % $C_7F_{15}H$

4

2,6 % $C_8F_{17}H$
< 0,001 % $C_7F_{15}I$
< 0,001 % $C_8F_{17}I$
97,3 % $C_8F_{17}Br$

Beispiel 8

Beispiel 7 wird wiederholt, wobei statt 15 g Polyethylenglykol 600 diesmal 15 g Polyethylenglykol 1000 PR eingesetzt werden. Die kapillargaschromatographische Untersuchung des rohen Perfluoroctylbromids nach der Umsetzung und vor der Destillation ergibt folgende Zusammensetzung:
0,074 % $C_7F_{15}H$
3,3 % $C_8F_{17}H$
< 0,001 % $C_7F_{15}I$
< 0,001 % $C_8F_{17}I$
96,6 % $C_8F_{17}Br$

Beispiel 9

In einem 50-ml-Glaskolben, ausgestattet mit Rückflußkühler, Magnetrührfisch, Innenthermometer und Tropftrichter mit Druckausgleich, werden 16,3 g Perfluoroctylbromid, das 0,63 % Perfluoroctyliodid und 0,017 % Perfluorheptyliodid enthält, vorgelegt und unter Stickstoff bei 60 °C gerührt. Eine Lösung von 0,4 g Natriumhydroxid in 7,5 g Polyethylenglykol 600 PR wird bei 50 bis 60 °C langsam über etwa 2 Stunden zugetropft. Danach wird weitere 6 Stunden bei 50 bis 60 °C gerührt. Von der Polyethylenglykol-600-PR-Phase werden während des Zutropfens und der Umsetzung kleine Proben aus der Reaktionsmischung zur pH-Kontrolle gezogen. Der pH-Wert wird zwischen 10 und 12 gehalten. Nach der Umsetzung wird bei Raumtemperatur Wasser zugegeben. Die obere, dunkelbraune Polyethylenglykol-600-PR/Wasser-Phase wird von der unteren, farblosen Perfluoroctylbromid-Phase abgetrennt und die Perfluoroctylbromid-Phase mehrmals mit destilliertem Wasser ausgeschüttelt. Die Untersuchung des rohen Perfluoroctylbromids vor der Destillation ergibt folgende Zusammensetzung:
0,011 % $C_7F_{15}H$
0,97 % $C_8F_{17}H$
< 0,001 % $C_7F_{15}I$
< 0,001 % $C_8F_{17}I$
98,9 % $C_8F_{17}Br$

Beispiele 10 bis 12

In einem 50-ml-Glaskolben, ausgestattet mit Rückflußkühler, Magnetrührfisch, Innenthermometer und Tropftrichter mit Druckausgleich, werden 30 g Perfluoroctylbromid zusammen mit Perfluoralkyliodid (siehe nachstehende Tabelle 2) vorgelegt. Eine Lösung von Base in Alkohol (siehe nachstehende Tabelle 2) wird langsam unter Rühren zugetropft. Nach der Umsetzung werden circa 20 ml Wasser zugegeben, die untere Perfluoroctylbromid-Phase von der oberen Alkohol-Wasser-Phase getrennt und die Perfluoroctylbromid-Phase mehrmals mit Wasser ausgeschüttelt. Das rohe Perfluoroctylbromid wird gaschromatographisch (gepackte Säule, Nachweisgrenze: 0,05 %) untersucht. Danach läßt man das rohe Perfluoroctylbromid langsam über eine Aktivkohlesäule laufen und reinigt das Eluat durch fraktionierende Destillation.

Tabelle 2

EP 0 678 495 A2

| Beispiel | Perfluoralkyliodid im Perfluoroctylbromid vor der Reaktion, Prozente bezogen auf Perfluoroctylbromid | Alkohol/Menge [g] | Base/Menge [g] | Reaktionstemperatur [°C] | Reaktionszeit [h] | Zusammensetzung des rohen Perfluoroctylbromids nach der Reaktion und vor der Destillation (GC) | |
|---|---|---|---|---|---|---|---|
| | | | | | | $C_8F_{17}H$ | $C_8F_{17}I$ |
| 10 | $C_8F_{17}I$ 1,9 % | Propy lenglykol/15 | NaOH 0,3 | 60 bis 69 | 8 | 2,8 % | < 0,05 % |
| 11 | $C_8F_{17}I$ 1,3 % | n-Pro panol/8 | KOH 0,8 | 50 | 6 | 3,5 % | < 0,05 % |
| 12 | $C_8F_{17}I$ 1,3 % | Ethanol/8 | KOH 0,8 | 50 | 4 | 3,3 % | < 0,05 % |

**Patentansprüche**

1. Verfahren zur Reinigung von Perfluorbromalkanen der Formel (I)

   $X\text{-}C_nF_{2n}\text{-}Br$      (I)

   in der X Fluor oder Brom und n eine Zahl von 2 bis 16 bedeuten, dadurch gekennzeichnet, daß man die zu reinigende Verbindung der Formel (I) mit einem primären oder sekundären Alkohol und einer Base unter guter Durchmischung in Kontakt bringt und die Alkohol-Base-Phase von der Verbindung der Formel (I) abtrennt.

2. Verfahren nach Anspruch 1, dadurch gekennzeichnet, daß ein Ausgangsmaterial der Formel (II)

   $Y\text{-}(CF_2)_m\text{-}Br$      (II)

   in der Y Fluor, Brom oder eine Gruppe der Formel $(CF_3)_2CF\text{-}$ und m eine Zahl von 2 bis 13 bedeuten, eingesetzt wird.

3. Verfahren nach Anspruch 1 oder 2, dadurch gekennzeichnet, daß ein Alkohol mit 2 bis 4 C-Atomen eingesetzt wird.

4. Verfahren nach Anspruch 1 oder 2, dadurch gekennzeichnet, daß als Alkohol ein Polyalkylenglykol eingesetzt wird.

5. Verfahren nach Anspruch 4, dadurch gekennzeichnet, daß das Polyalkylenglykol ein Polyethylenglykol oder ein Polypropylenglykol ist.

6. Verfahren nach Anspruch 5, dadurch gekennzeichnet, daß das Polyalkylenglykol eine mittlere Molmasse von 200 bis 2000 hat.

7. Verfahren nach einem oder mehreren der vorhergehenden Ansprüche, dadurch gekennzeichnet, daß das Masseverhältnis der Verbindung der Formel (I) zum Alkohol zwischen 20 : 1 und 1 : 1 liegt.

8. Verfahren nach einem oder mehreren der vorhergehenden Ansprüche, dadurch gekennzeichnet, daß die Base ein Alkalimetallalkoholat, Alkalimetalloxid oder Alkalimetallhydroxid ist.

9. Verfahren nach einem oder mehreren der vorhergehenden Ansprüche, dadurch gekennzeichnet, daß die von der Alkohol-Base-Phase abgetrennte gereinigte Verbindung der Formel (I) einem oder mehreren der Verfahrensschritte Waschen mit Wasser, Behandeln mit Aktivkohle sowie Destillation unterworfen wird.

10. Verfahren nach einem oder mehreren der vorhergehenden Ansprüche, dadurch gekennzeichnet, daß die Umsetzung bei -10 bis +150 °C erfolgt.